# EUROPEAN PATENT SPECIFICATION

(11) **EP 1 677 761 B1**
(45) Date of publication and mention of the grant of the patent: **09.04.2008**
(21) Application number: 04810041.6
(22) Date of filing: 27.10.2004
(51) Int. Cl.: A61K 9/10, A61K 31/56, A61K 31/7036

(54) **SUSPENSION OF LOTEPREDNOL ETABONATE AND TOBRAMYCIN FOR TOPICAL OPHTHALMIC USE**
SUSPENSION VON LOTEPREDNOL-ETABONAT UND TOBRAMYCIN FÜR DIE TOPISCHE OPHTHALMISCHE ANWENDUNG
SUSPENSION D'ETABONATE DE LOTEPREDNOL ET DE TOBRAMYCINE POUR UTILISATION OPHTALMIQUE TOPIQUE

(30) Priority: 31.10.2003 US 698322
(43) Date of publication of application: 12.07.2006
(73) Proprietor: BAUSCH & LOMB INCORPORATED, Rochester, New York 14604 (US)
(72) Inventor: KRISHNAMOORTHY, Ramesh, Cary, NC 27519 (US)
(74) Representative: Maiwald Patentanwalts GmbH
(86) International application number: PCT/US2004/035562
(87) International publication number: WO 2005/044231

(56) References cited:
- WO-A-95/11669
- WO-A-20/05000317
- US-A1- 2002 098 154

## Description

### FIELD OF THE INVENTION

This invention relates to formulations for topical use comprising antibiotics in combination with anti-inflammatory steroids for treating ophthalmic infections and attendant inflammation. More specifically, this invention relates to pharmaceutical ophthalmic formulations comprising a pH stabilizing amount of tobramycin and the soft steroid loteprednol etabonate.

### BACKGROUND OF THE INVENTION

Topical steroids such as corticosteroids are commonly used for anti-inflammatory therapy of the eye, especially for treating inflammatory conditions of the palpebral or bulbar conjunctiva, cornea and anterior segment of the globe. Common therapeutic applications for steroids include allergic-conjunctivitis, acne rosacea, superficial punctate keratitis and iritis cyclitis. Steroids also are used to ameliorate inflammation associated with corneal injury due to chemical or thermal burns, or penetration of foreign bodies. Such conditions may result from surgery, injury, allergy or infection to the eye and can cause severe discomfort.

Despite their therapeutic advantages, topical ocular use of corticosteroids is associated with a number of complications, including posterior subcapsular cataract formation, elevation of intraocular pressure, secondary ocular infection, retardation of corneal wound healing, uveitis, mydriasis, transient ocular discomfort and ptosis. Numerous systemic complications also may arise from the topical ocular application of corticosteroids. These complications include adrenal insufficiency, Cushing's syndrome, peptic ulceration, osteoporosis, hypertension, muscle weakness or atrophy, inhibition of growth, diabetes, activation of infection, mood changes and delayed wound healing.

Topical steroids for treating ocular inflammations can be based on soft drugs. Soft drugs, as is known in the art, are designed to provide maximal therapeutic effect and minimal side effects. By one approach, synthesis of a "soft drug" can be achieved by structurally modifying a known inactive metabolite of a known active drug to produce an active metabolite that undergoes a predictable one-step transformation in-vivo back to the parent, inactive metabolite (see, U.S. Pat. Nos. 6,610,675, 4,996,335 and 4,710,495 for soft steroids). "Soft drugs" therefore are biologically active chemical components characterized by predictable in vivo metabolism to non-toxic derivatives after they provide their therapeutic effect. Formulations of cortico steroids suitable for ophthalmic use are known. For example, US patents 4,710,495, 4,996,335, 5,540,930, 5,747,061, 5,916,550, 6,368,616 and 6,610,675, describe soft steroids and formulations containing soft steroids.

Antibiotic agents for use in treating ophthalmic infections are also known. For example penicillins, cephalosporins and aminoglycosides such as amikacin, gentamicin and tobramycin are known to be useful in treating infections of the eye. Tobramycin is commercially marketed and well recognized as an effective antibiotic. This particular anti-infective is recognized as active against the common bacterial eye pathogens: Staphylococci, including *S. aureus* and *S. epidermidis,* including penicillin resistant strains, Streptococci, including *S. pneumoniae, Pseudomonas aeruginosa, Escherichia coli, Klebsiella pneumoniae, Enterobacter aerogenes, Proteus mirabilis, Morganella morganii, Haemophilus influenzae, H. aegyptius, Acinetobacter calcoaceticus* and some *Neissaria* species. Tobramycin's antimicrobial activity is provided by its ability to bind with the 30S ribosomal subunit and alter protein synthesis, thus leading to the death of the microbial organism.

It has previously been suggested that the steroid loteprednol etabonate (LE) can be combined with antibiotics such as tobramycin. However, there has been no suggestion of the amount of tobramycin to be used in combination with LE to provide a desired therapeutic effect of both active agents. There has also been no detailed description of a combined formulation having satisfactory properties for storage and use of the combination of tobramycin and LE.

It is known that formulations containing steroids can experience stability problems. Such stability problems include clumping and other undesirable changes upon storage. U.S. Patent No. 5,916,550 describes the use of C₂-C₇ aliphatic amino acids to control pH depression of aqueous suspensions of LE on prolonged storage. Therefore, the need to provide pharmaceutical formulations of steroids that are stable upon storage is well recognized. One of the factors used to evaluate stability of pharmaceutical formulations is pH. When there is a dramatic change in the pH of a pharmaceutical formulation over time, the ability of the formulation to be effectively stored and retain its pharmaceutical activity after storage becomes questionable. It is known to add buffers to certain pharmaceutical formulations in an effort to maintain the stability of the formulation during storage. Examples of buffers include borate buffers, phosphate buffers, etc. Although these buffers are useful in stabilizing pH, they do not demonstrate pharmaceutical activity. Therefore, it would be desirable to use a single material to provide pH stabilizing activity and desired anti-infection activity to pharmaceutical ophthalmic formulations containing the soft steroid loteprednol etabonate.

### SUMMARY OF THE INVENTION

It has surprisingly been discovered that the aminoglycoside tobramycin, when present in a pH stabilizing amount, helps to stabilize loteprednol etabonate containing formulations over time to provide better storage characteristics.

This invention provides novel compositions of matter containing a combination of water-soluble and water-insoluble drugs suitable for therapeutic use. The invention provides pH stable aqueous suspensions of water-insoluble drugs that remain in such a state even after extended periods of storage.

More particularly, the invention is directed to aqueous suspensions of soft steroids such as loteprednol etabonate in combination with aminoglycosides such as tobramycin suitable for therapeutic use in the eye, ear, or nose. The aqueous suspensions of LE and tobramycin are surprisingly pH stable and can remain in a pH stable state for extended periods of storage.

The present invention refers to a composition for ophthalmic or otolaryngological anti-inflammatory use as defined in independent claim 1. According to another aspect of the present invention, the inventive composition is used for the manufacture of a medicament for treating ophthalmic or otolaryngological inflammation and infection.

Formulations comprising the broad spectrum aminoglycoside antibiotic tobramycin in combination with the predictably metabolized steroid loteprednol etabonate provide pharmaceutical ophthalmic formulations that not only allow for the simultaneous treatment of inflammation and infection in a patient in need of treatment thereof, but also results in a pharmaceutical ophthalmic formulation having increased stability, as measured by decreased change in pH as compared to similar formulations that do not contain tobramycin.

Further provided herein is a topical eye drop medication indicated for steroid-responsive inflammatory ocular conditions for which a corticosteroid is indicated and where superficial bacterial ocular infection or risk of bacterial ocular infection exists. The medication comprises a loteprednol etabonate/tobramycin ophthalmic suspension, 0.5%/0.3%. The use of this medication is indicated where the risk of superficial ocular infection is high or where there is an expectation that potentially dangerous numbers of bacteria will be present in the eye.

Also provided herein is a therapeutically effective composition comprising a soft steroid such as loteprednol etabonate in an amount effective to provide a therapeutic benefit to a patient to whom the composition is administered and a pH stabilizing amount of tobramycin, wherein the tobramycin is present in an amount effective to stabilize the pH of the composition relative to the pH of a similar formulation without the tobramycin.

Also provided herein is a method of treating a patient having inflammatory ocular conditions for which a corticosteroid is indicated and where superficial bacterial ocular infection or risk of bacterial ocular infection exists, the method comprising topically applying to a patient in need of treatment thereof a therapeutic amount of a pharmaceutical composition comprising the broad spectrum aminoglycoside antibiotic tobramycin in combination with the predictably metabolized steroid loteprednol etabonate.

Having briefly summarized the invention, the invention will now be described in detail by reference to the following specification and examples. Unless otherwise specified, all percentages are by weight and all temperatures are in degrees Celsius.

### DETAILED DESCRIPTION OF THE INVENTION

Therapeutic suspensions of LE for ophthalmic or otolaryngological uses are made by aseptic preparation. Purity levels of all materials employed in the suspensions of the invention exceed 98%. The suspensions of the invention are prepared by thoroughly mixing the soft steroid (component (A)), aminoglycoside (component (B)), suspending agent (component (C)), and surface active agent (component (D)). Optionally, tonicity agents (component (E)) and preservatives (component (F)) may be included.

Steroids of component (A), preferably soft steroids, most preferably LE, can be employed. Also other steroids such as beclomethasone, betamethasone, fluocinolone, fluorometholone, exednisolone, may be employed. The suspensions of component (A) of the invention have a particle size of 0.1-30 µ, preferably 1-20 µ, most preferably 2-10 microns in mean diameter. LE in this size range is commercially available from suppliers such as the Sipsy Co., (Avrille, France).

The aminoglycoside component (B) is pharmaceutical grade. Aminoglycosides are a well-characterized family of antimicrobial agents and include, for example, gentamicin, neomycin paromomycin, kanamycin, tobramycin, netilmicin and amikacin. Tobramycin of this grade is commercially available from suppliers such as the Biogal Pharmaceutical Works (Debrecen, Hungary). Component (B) is preferably present in an amount that is effective to stabilize the pH of the composition relative to the pH of a similar composition without Component (B). Therefore the amount of Component (B) may vary depending upon the individual composition. Determining a pH stabilizing amount of an aminoglycoside for a particular composition can be readily achieved through routine experimentation and is within the purview of one skilled in the art.

The nonionic polymer of component (C) can be any nonionic water-soluble polymer. Typical compounds such as PVP, PVA, HPMC or dextran can be used at a concentration of 0.01-2%, and preferably between 0.4 to 1.5%, and more preferably between 0.4 to 1%. Viscosity increased above that of simple aqueous solutions may be desirable to increase ocular absorption of the active compound, to decrease variability in dispensing the formulation, to decrease physical separation of components of a suspension or emulsion of the formulation and/or to otherwise improve the ophthalmic formulation. Such viscosity builder agents include as examples polyvinyl alcohol, polyvinyl pyrrolidone, methyl cellulose, hydroxy propyl methylcellulose, hydroxyethyl cellulose, carboxymethyl cellulose, hydroxy propyl cellulose or other agents known to those skilled in the art. Povidone is preferably used as a suspending agent in the finished product and the water-soluble grades are routinely used in pharmaceuticals as a viscosity enhancing agent. The viscosity of aqueous solutions of the water-soluble grades of povidone depends on the average molecular weight. A subtle change in the grade and concentration of the suspending agent can yield the desired characteristics. Povidone comes in a variety of grades, of which some are water soluble. Povidone^{®} K-90 is the highest molecular weight water-soluble viscosity grade Povidone. This material is listed as Povidone, USP 90,000. The high molecular grade povidone dissolves much more slowly than the lower molecular weight grade.

Component (D) is a surface-active agent that is acceptable for ophthalmic or otolaryngological uses. Preferably, this surfactant is non-ionic. Useful surface active agents include but are not limited to polysorbate 80, tyloxapol, TWEEN 80^{®} (ICI America Inc., Wilmington, Del.), PLURONIC F-68^{®} (from BASF, Ludwigshafen, Germany) and the poloxamer surfactants. These surfactants are nonionic alkaline oxide condensates of an organic compound that contains hydroxyl groups. The concentration in which the surface active agent may be used is only limited by neutralization of the bactericidal effects on the accompanying preservatives, or by concentrations that may cause irritation. Preferably, the concentration of component (D) is 0.05 to 1%, and more preferably 0.1 to 0.6% by weight based on the weight of the suspension. Composition of the present invention having a molar ratio of (A):(C):(D) between 1:20:1 and 1:0.01:0.5 are entirely suitable.

The tonicity agents of component (E) can be nonionic diols, preferably glycerol, in sufficient amounts to achieve isotonicity. The nonionic tonicity agents can be present in an amount of about 2 to 2.8% by weight, and preferably about 2.2 to 2.6%.

The nonionic polymeric compounds of component (C), and the surface active agents of component (D) have good solubility in water, have sufficient number of hydroxyl groups to interact with the steroid, and have mild effects on the viscosity of the suspension. Final viscosity should not exceed 80-centipoise.

The suspensions of the invention also may include additional therapeutic drugs such as drugs for treating glaucoma, anti-inflammatory drugs, anti-cancer drugs, antifungal drugs and anti-viral drugs. Examples of anti-glaucoma drugs include but are not limited to timolol-base, betaxalol, athenolol, levobanolol, epinenephrin, dipivalyl, oxonolol, acetazilumide-base and methazalomide. Examples of anti-inflammatory drugs include but are not limited to non-steroids such as piroxicam, indomethacin, naproxen, phenylbutazone, ibuprofen and diclofenac.

Health regulations in various countries generally require that ophthalmic preparations shall include a preservative. Many well known preservatives that have been used in ophthalmic preparations of the prior art, however, cannot be used in the preparations of the invention, since those preservatives may no longer be considered safe for ocular use, or may interact with the surfactant employed in the suspension to form a complex that reduces the bactericidal activity of the preservative.

The preservatives of component (F) employed in the suspensions of the invention therefore are chosen to not interact with the surface active agent to an extent that the preservatives are prevented from protecting the suspension from microbiological contamination. In a preferred embodiment benzalkonium chloride may be employed as a safe preservative, most preferably benzalkonium chloride with EDTA. Other possible preservatives include but are not limited to benzyl alcohol, methyl parabens, propyl parabens, thimerosal, chlorbutanol and benzethonium chlorides. Typically such preservatives are employed at a level of from 0.001% to 1.0% by weight. Preferably, a preservative (or combination of preservatives) that will impart standard antimicrobial activity to the suspension and protect against oxidation of components (A)-(E) is employed.

In forming compositions for topical administration, the mixtures are preferably formulated as 0.01 to 2.0 percent by weight solutions in water at a pH of 4.5 to 8.0 (figures relate to combined presence of loteprednol etabonate and tobramycin). While the precise regimen is left to the discretion of the clinician, it is recommended that the resulting solution be topically applied by placing one drop in each eye two times a day.

A bioavailability study of LE-tobramycin vs. LOTEMAX loteprednol etabonate composition demonstrated that in the intend to treat population bioequivalence was met at both the 40 and 60 minute sampling periods. Thus, the inclusion of tobramycin does not alter the ocular bioavailability of loteprednol etabonate. A microbial kill rate study was undertaken to demonstrate antimicrobial equivalence between loteprednol etabonate and tobramycin ophthalmic suspension, 0.5%/0.3% and tobramycin ophthalmic solution, USP 0.3%. The methods employed were based on USP anti-microbial effectiveness procedures for preparation of inoculum and challenge concentration of test organisms. The anti-microbial activity of both products was demonstrated against 22 organisms. The *in vitro* study demonstrated that tobramycin has equivalent anti-microbial activity as a single agent and when in combination with loteprednol etabonate.

Without further elaboration, it is believed that one skilled in the art can, using the preceding description, utilize the present invention to its fullest extent. In the following examples, all temperatures are set forth in degrees Celsius; unless otherwise indicated, all parts and percentages are by weight.

### EXAMPLES

A study was undertaken to compare a standard LE-tobramycin composition having different concentrations of Povidone and different types of Povidone. The example compositions also contained standard pharmaceutical components. Examples III and VI were used as controls (no tobramycin) to observe the effect of tobramycin on the pH of the composition. The materials were mixed with purified water and held at a temperature of 28 °C to represent room temperature stability and 40 °C to represent accelerated stability. The results are given in the tables below.

### STABILITY OF LE-TOBRAMYCIN MATRIX (with different viscosity)

### 28 °C

| | Time | Tobra. | LE | |
|---|---|---|---|---|
| | (month) | (mg/ml) | (mg/ml) | pH |
| I | 0 | 3.18 | 4.976 | 6.49 |
| 0.6% | 1 | 3.076 | -- | 6.32 |
| PVP-C30 | 2 | 3.062 | 4.947 | 6.28 |
| | 3 | 3.113 | 5.484 | 6.21 |
| | 6 | 3.003 | 5.155 | 6.17 |
| II | 0 | 3.165 | 5.241 | 6.47 |
| 1.5% | 1 | 3.04 | -- | 6.3 |
| PVP-C30 | 2 | 2.995 | 5.08 | 6.19 |
| | 3 | 3.04 | 5.32 | 6.14 |
| | 6 | 3.008 | 5.514 | 6.087 |
| III | 0 | -- | 5.576 | 5.98 |
| 1.5% | 1 | -- | -- | 5.54 |
| PVP-C30 | 2 | -- | 5.411 | 5.06 |
| Control | 3 | -- | 5.706 | 5.02 |
| | 6 | -- | 5.134 | 4.8 |
| IV | 0 | 3.206 | 5.296 | 6.71 |
| 1.5% | 1 | 3.082 | 5.156 | 6.57 |
| PVP-K90 | 2 | 3.122 | 5.29 | 6.49 |
| | 3 | 3.21 | 5.306 | 6.47 |
| | 6 | 3.146 | 5.358 | 6.39 |
| V | 0 | 2.802 | 4.32 | 6.61 |
| 0.5% | 1 | 2.754 | 4.24 | 6.48 |
| PVP-K90 | 2 | 2.596 | 4.28 | 6.38 |
| | 3 | 2.811 | 4.336 | 6.34 |
| | 6 | 2.806 | 4.365 | 6.32 |
| VI | 0 | -- | 5.426 | 6.61 |
| 1.5% | 1 | -- | 5.638 | 5.033 |
| PVP-K90 | 2 | -- | 5.67 | 4.71 |
| Control | 3 | -- | 5.67 | 4.72 |
| | 6 | -- | 5.727 | 4.43 |

### STABILITY OF LE-TOBRAMYCIN MATRIX (with different viscosity)

### 40 °C

| | Time | Tobra. | LE | |
|---|---|---|---|---|
| | (month) | (mg/ml) | (mg/ml) | pH |
| I | 0 | 3.18 | 4.976 | 6.49 |
| 0.6% | 1 | 3.15 | -- | 6.23 |
| PVP-C30 | 2 | 2.957 | 5.008 | 6.06 |
| | 3 | 3.036 | 5.067 | 5.95 |
| | 6 | | | |
| II | 0 | 3.165 | 5.241 | 6.47 |
| 1.5% | 1 | 3.019 | -- | 6.21 |
| PVP-C30 | 2 | 2.91 | 5.16 | 5.89 |
| | 3 | 2.95 | 5.37 | 5.89 |
| | 6 | | | |
| III | 0 | -- | 5.576 | 5.98 |
| 1.5% | 1 | -- | -- | 4.73 |
| PVP-C30 | 2 | -- | 5.411 | 4.33 |
| Control | 3 | -- | 5.473 | 4.11 |
| | 6 | -- | | |
| IV | 0 | 3.206 | 5.296 | 6.71 |
| 1.5% | 1 | 2.94 | 5.336 | 6.47 |
| PVP-K90 | 2 | 2.84 | 5.209 | 6.13 |
| | 3 | 3.17 | 5.17 | 6.25 |
| | 6 | 3.212 | 5.178 | 6.097 |
| V | 0 | 2.802 | 4.32 | 6.61 |
| 0.5% | 1 | 2.588 | 4.284 | 6.38 |
| PVP-K90 | 2 | 2.64 | 4.21 | 6.21 |
| | 3 | 2.82 | 4.2 | 6.14 |
| | 6 | 2.767 | 4.267 | 6.04 |
| VI | 0 | -- | 5.426 | 6.61 |
| 1.5% | 1 | -- | 5.614 | 4.62 |
| PVP-K90 | 2 | -- | 5.91 | 3.93 |
| Control | 3 | -- | 5.85 | 3.83 |
| | 6 | -- | 5.572 | 3.53 |

The above data represents the results of pH stability testing of various compositions having differing viscosity. PVP-C30 is Povidone having a molecular weight of around 30,000 and PVP-K90 is Povidone having a molecular weight of around 90,000. Both were obtained from the GAF Corporation, USA. In general a pH between 4.5 and 7.0 is considered acceptable for pharmaceutical ophthalmologic use of these compositions. The data demonstrates that compositions of the present invention having tobramycin display a more gradual decrease in pH over time and less of a total change in pH over time as compared to similar compositions which do not contain tobramycin.

The following example is a representative pharmaceutical composition of the invention for topical use where indicated against inflammation and infection.

### EXAMPLE 1

### Ingredients (per mL)

Loteprednol etabonate 0.5% (5 mg)
Glycerin 2.5%
Povidone, K-90 0.6%
Tobramycin 0.3% (3 mg)
Benzalkonium Chloride 0.01%
Tyloxapol 0.05%
Edentate disodium 0.01%
Purified water (QS to 100%)
Sulfuric acid or sodium hydroxide (to adjust pH)

## Claims

1. A composition for ophthalmic or otolaryngological anti-inflammatory use comprising:
(A) loteprednol etabonate having a particle size of 0.1 to 30 microns in diameter in an amount of 0.2 to 2 % by weight;
(B) tobramycin in an amount effective to stabilize the pH of the composition relative to the pH of a similar composition without the tobramycin;
(C) a nonionic polymer in an aqueous medium; and
(D) a nonionic surface active agent in an amount sufficient to retain the loteprednol etabonate in suspension, wherein the molar ratio of(A):(C):(D) is between 1:20:1 and 1:0.01:0.5.

2. The composition of claim 1 further comprising:
(E) a nonionic tonicity agent in an amount sufficient to achieve isotonicity.

3. The composition of claim 1, wherein the loteprednol etabonate is present in an amount of 0.5 to 1 % by weight.

4. The composition of claim 1, wherein the loteprednol etabonate has a particle size less than about fifteen microns.

5. The composition of claim 4, wherein the tobramycin is present in an effective anti-infection amount.

6. The composition of claim 1 further including a preservative for preventing microbial formation in said composition in an amount of 0.01 to 0.025% by weight.

7. The composition of claim 6, wherein said preservative is benzalkonium chloride.

8. The composition of claim 7 further comprising disodium edetate.

9. The composition of claim 1, wherein said nonionic polymer is selected from the group consisting of polyvinylpyrrolidone, polyvinyl alcohol, or dextran and is present in an amount of 0.2 to 2 % by weight and wherein the nonionic surface active agent is present in an amount of 0.05 to 1 % by weight.

10. The composition of claim 1, wherein said nonionic polymer is polyvinylpyrrolidone and is present in an amount of 0.4 to 1 % by weight.

11. The compositions of claim 1, wherein said nonionic surface active agent is cyloxapol and is present in an amount of 0.1 to 0.6 % by weight.

12. The composition of claim 1 further comprising an additional therapeutic drug in admixture with loteprednol etabonate and tobramycin, wherein said additional therapeutic drug is selected from the group consisting of betaxolol, atenolol, levobunolol, epinephrine, dipivalyl, oxonolol, acetazolamide-base, methazolamide, piroxicam, indomethacin, naproxen, phenylbutazone, ibuprofen, and diclofenac-acid.

13. The composition of claim 2, wherein the loteprednol etabonate is present in an amount of between 0.01 and 1 % by weight.

14. The composition of claim 2, wherein the loteprednol etabonate is present in an amount of between 0.05 and 0.5 % by weight.

15. The composition of claim 2, wherein said nonionic polymer is a water soluble polymer selected from the group consisting of polyvinylpyrrolidone, polyvinyl alcohol, dextran and cyclodextrin.

16. The composition of claim 2, wherein the non ionic polymer is present in an amount of 0.2 to 2 % by weight.

17. The composition of claim 15, wherein said nonionic polymer is polyvinylpyrrolidone and is present in an amount of between 0.3 to 1.75 % by weight.

18. The composition of claim 2, wherein said nonionic surface active agent is tyloxapol and is present in an amount of 0.05 to 1 % by weight.

19. The composition of claim 2, wherein said nonionic surface active agent is a polyoxyethylene sorbitan mono-oleate ester.

20. The composition of claim 17, wherein the nonionic tonicity agent is present in an amount of between 1 to 7 % by weight.

21. The composition of claim 20, wherein the nonionic tonicity agent is a nonionic polyol and is present in an amount of between 1.5 to 4 % by weight.

22. The composition of claim 21, wherein the nonionic tonicity agent is glycerol or mannitol.

23. The composition of claim 2 further including a preservative for preventing microbial formation in said composition and is present in an amount of between 0.0001 to 0.025 % by weight.

24. The composition of claim 23, wherein said preservative is selected from the group consisting of benzalkonium chloride, disodium edetate and mixtures thereof.

25. The composition of claim 2 further comprising an additional therapeutic drug in admixture with loteprednol etabonate and tobramycin, wherein said additional therapeutic drug is selected from the group consisting of betaxolol, atenolol, levobunolol, epinephrine, dipivalyl, oxonolol, acetazolamide-base, methazolamide, piroxicam, indomethacin, naproxen, phenylbutazone, ibuprofen, and diclofenac.

26. The composition of claim 2, wherein the nonionic polymer is present in an amount of between 0.2 to 2 % by weight; the nonionic tonicity agent is present in an amount of between 1 to 7 % by weight; and the nonionic surface active agent is present in an amount of between 0.05 to 1 % by weight.

27. The composition of claim 26 further comprising a preservative for preventing microbial formation in said composition and is present in an amount of 0.0001 to 0.025 by weight.

28. Use of a composition comprising:
(A) loteprednol etabonate in an amount of 0.01 to about 2% by weight and having a particle size of 0.1 to 30 microns in diameter;
(B) an amount of tobramycin effective to stabilize the pH of the composition relative to the pH of a similar composition without the tobramycin;
(C) a nonionic polymer in an aqueous medium; and
(D) a nonionic surface active agent in an amount sufficient to retain the loteprednol etabonate in suspension, wherein the molar ratio of (A):(C):(D) is between 1:20:1 and 1:0.01:0.5 for the manufacture of a medicament for treating ophthalmic or otolaryngological inflammation and infection.

## Patentansprüche

1. Eine Zusammensetzung zur ophtalmischen oder otolaryngologischen anti-entzündlichen Verwendung, umfassend:
(A) Loteprednol-Etabonat mit einer Partikelgröße von 0,1 bis 30 Mikrometer im Durchmesser in einer Menge von 0,2 bis 2 Gew.-%;
(B) Tobramycin in einer Menge, die den pH-Wert der Zusammensetzung im Vergleich zu dem pH-Wert einer ähnlichen Zusammensetzung ohne Tobramycin wirksam stabilisiert;
(C) ein nichtionisches Polymer in einem wässrigen Medium; und
(D) ein nichtionischer oberflächenaktiver Stoff in einer Menge, die ausreicht, um das Loteprednol-Etabonat in Suspension zu halten, wobei das molare Verhältnis von (A):(C):(D) zwischen 1:20:1 und 1:0,01:0,5 liegt.

2. Die Zusammensetzung gemäß Anspruch 1, weiter umfassend:
(E) ein nichtionisches Tonizitätsmittel in einer Menge, die ausreicht, um Isotonizität zu erreichen.

3. Die Zusammensetzung gemäß Anspruch 1, wobei das Loteprednol-Etabonat in einer Menge von 0,5 bis 1 Gew.-% vorhanden ist.

4. Die Zusammensetzung gemäß Anspruch 1, wobei das Loteprednol-Etabonat eine Partikelgröße von weniger als etwa 15 Mikrometern aufweist.

5. Die Zusammensetzung gemäß Anspruch 4, wobei das Tobramycin in einer wirksamen anti-infektiösen Menge vorhanden ist.

6. Die Zusammensetzung gemäß Anspruch 1, weiter beinhaltend ein Konservierungsmittel in einer Menge von 0,01 bis 0,025 Gew.-% zur Vermeidung mikrobieller Entwicklung in besagter Zusammensetzung.

7. Die Zusammensetzung gemäß Anspruch 6, wobei besagtes Konservierungsmittel Benzalkoniumchlorid ist.

8. Die Zusammensetzung gemäß Anspruch 7, weiter umfassend Dinatriumedetat.

9. Die Zusammensetzung gemäß Anspruch 1, wobei besagtes nichtionisches Polymer ausgewählt ist aus der Gruppe bestehend aus Polyvinylpyrrolidon, Polyvinylalkohol oder Dextran und in einer Menge von 0,2 bis 2 Gew.-% vorhanden ist, und wobei der nichtionische oberflächenaktive Stoff in einer Menge von 0,05 bis 1 Gew.-% vorhanden ist.

10. Die Zusammensetzung gemäß Anspruch 1, wobei besagtes nichtionisches Polymer Polyvinylpyrrolidon ist und in einer Menge von 0,4 bis 1 Gew.-% vorhanden ist.

11. Die Zusammensetzung gemäß Anspruch 1, wobei besagter nichtionischer oberflächenaktiver Stoff Tyloxapol ist und in einer Menge von 0,1 bis 0,6 Gew.-% vorhanden ist.

12. Die Zusammensetzung gemäß Anspruch 1, weiter umfassend einen zusätzlichen therapeutischen Wirkstoff in Mischung mit Loteprednol-Etabonat und Tobramycin, wobei besagter zusätzlicher therapeutischer Wirkstoff ausgewählt ist aus der Gruppe bestehend aus Betaxolol, Atenolol, Levobunolol, Epinephrin, Dipivalyl, Oxonolol, Acetazolamidbase, Methazolamid, Piroxicam, Indomethacin, Naproxen, Phenylbutazon, Ibuprofen und Diclofenacsäure.

13. Die Zusammensetzung gemäß Anspruch 2, wobei das Loteprednol-Etabonat in einer Menge zwischen 0,01 bis 1 Gew.-% vorhanden ist.

14. Die Zusammensetzung gemäß Anspruch 2, wobei das Loteprednol-Etabonat in einer Menge zwischen 0,05 und 0,5 Gew.-% vorhanden ist.

15. Die Zusammensetzung gemäß Anspruch 2, wobei besagtes nichtionisches Polymer ein wasserlösliches Polymer ist, ausgewählt aus der Gruppe bestehend aus Polyvinylpyrrolidon, Polyvinylalkohol, Dextran und Cylodextrin.

16. Die Zusammensetzung gemäß Anspruch 2, wobei das nichtionische Polymer in einer Menge von 0,2 bis 2 Gew.-% vorhanden ist.

17. Die Zusammensetzung gemäß Anspruch 15, wobei besagtes nichtionisches Polymer Polyvinylpyrrolidon ist und in einer Menge zwischen 0,3 bis 1,75 Gew.-% vorhanden ist.

18. Die Zusammensetzung gemäß Anspruch 2, wobei besagter nichtionischer oberflächenaktiver Stoff Tyloxapol ist und in einer Menge von 0,05 bis 1 Gew.-% vorhanden ist.

19. Die Zusammensetzung gemäß Anspruch 2, wobei besagter nichtionischer oberflächenaktiver Stoff ein Polyoxyethylensorbitanmonooleatester ist.

20. Die Zusammensetzung gemäß Anspruch 17, wobei das nichtionische Tonizitätsmittel in einer Menge zwischen 1 bis 7 Gew.-% vorhanden ist.

21. Die Zusammensetzung gemäß Anspruch 20, wobei das nichtionische Tonizitätsmittel ein nichtionisches Polyol ist und in einer Menge von zwischen 1,5 bis 4 Gew.-% vorhanden ist.

22. Die Zusammensetzung gemäß Anspruch 21, wobei das nichtionische Tonizitätsmittel Glycerol oder Mannitol ist.

23. Die Zusammensetzung gemäß Anspruch 2, weiter umfassend ein Konservierungsmittel zur Vermeidung mikrobieller Entwicklung in besagter Zusammensetzung, wobei dieses in einer Menge zwischen 0,0001 bis 0,025 Gew.-% vorhanden ist.

24. Die Zusammensetzung gemäß Anspruch 23, wobei besagtes Konservierungsmittel ausgewählt ist aus der Gruppe bestehend aus Benzalkoniumchlorid, Dinatriumedetat und Mischungen davon.

25. Die Zusammensetzung gemäß Anspruch 2, weiter umfassend einen zusätzlichen therapeutischen Wirkstoff in Mischung mit Loteprednol-Etabonat und Tobramycin, wobei besagter zusätzlicher therapeutischer Wirkstoff ausgewählt ist aus der Gruppe bestehend aus Betaxolol, Atenolol, Levobunolol, Epinephrin, Dipivalyl, Oxonolol, Acetazolamidbase, Methazolamid, Piroxicam, Indomethacin, Naproxen, Phenylbutazon, Ibuprofen und Dicolfenac.

26. Die Zusammensetzung gemäß Anspruch 2, wobei das nichtionische Polymer in einer Menge zwischen 0,2 bis 2 Gew.-% vorhanden ist; das nichtionische Tonizitätsmittel in einer Menge zwischen 1 bis 7 Gew.-% vorhanden ist und der nichtionische oberflächenaktive Stoff in einer Menge zwischen 0,05 bis 1 Gew.-% vorhanden ist.

27. Die Zusammensetzung von Anspruch 26, weiter umfassend ein Konservierungsmittel zur Vermeidung mikrobieller Entwicklung in besagter Zusammensetzung, wobei dieses in einer Menge von 0,00001 bis 0,025 Gew.-% vorhanden ist.

28. Verwendung einer Zusammensetzung, umfassend:
(A) Loteprednol-Etabonat in einer Menge von 0,01 bis etwa 2 Gew.-% und mit einer Partikelgröße von 0,1 bis 30 Mikrometern im Durchmesser;
(B) eine Menge von Tobramycin, die den pH-Wert der Zusammensetzung im Vergleich zu dem pH-Wert einer ähnlichen Zusammensetzung ohne Tobramycin wirksam stabilisiert;
(C) ein nichtionisches Polymer in einem wässrigen Medium; und
(D) ein nichtionischer oberflächenaktiver Stoff in einer Menge, die ausreicht, um das Loteprednol-Etabonat in Suspension zu halten, wobei das Molverhältniss von (A):(C):(D) zwischen 1:20:1 und 1:0,01:0,5 für die Herstellung eines Medikaments zur Behandlung einer ophtalmischer oder otolaryngologischer Entzündung und Infektion liegt.

## Revendications

1. Composition pour une utilisation anti-inflammatoire ophtalmique ou oto-rhino-laryngologique comprenant :
(A) de l'étabonate de lotéprednol ayant une taille de particule de 0,1 à 30 microns de diamètre en une quantité de 0,2 à 2 % en poids ;
(B) de la tobramycine en une quantité efficace pour stabiliser le pH de la composition par rapport au pH d'une composition similaire sans tobramycine ;
(C) un polymère non ionique dans un milieu aqueux ; et
(D) un agent tensioactif non ionique en une quantité suffisante pour garder l'étabonate de lotéprednol en suspension, dans laquelle le rapport molaire de (A) : (C) : (D) est compris entre 1 : 20 : 1 et 1 : 0,01 : 0,5.

2. Composition selon la revendication 1, comprenant en outre :
(E) un agent de tonicité en une quantité suffisante pour atteindre l'isotonie.

3. Composition selon la revendication 1, dans laquelle l'étabonate de lotéprednol est présent en une quantité de 0,5 à 1 % en poids.

4. Composition selon la revendication 1, dans laquelle l'étabonate de lotéprednol a une taille de particule inférieure à environ quinze microns.

5. Composition selon la revendication 4, dans laquelle la tobramycine est présente en une quantité anti-infection efficace.

6. Composition selon la revendication 1, comprenant en outre un conservateur pour empêcher la formation microbienne dans ladite composition en une quantité de 0,01 à 0,025 % en poids.

7. Composition selon la revendication 6, dans laquelle ledit conservateur est le chlorure de benzalkonium.

8. Composition selon la revendication 7, comprenant en outre de l'édetate de disodium.

9. Composition selon la revendication 1, dans laquelle ledit polymère non ionique est choisi dans le groupe constitué par la polyvinylpyrrolidone, l'alcool polyvinylique ou le dextran et est présent en une quantité de 0,2 à 2 % en poids et dans laquelle l'agent tensioactif non ionique est présent en une quantité de 0,05 à 1 % en poids.

10. Composition selon la revendication 1, dans laquelle ledit polymère non ionique est la polyvinylpyrrolidone et est présent en une quantité de 0,4 à 1 % en poids.

11. Composition selon la revendication 1, dans laquelle ledit agent tensioactif non ionique est le tyloxapol et est présent en une quantité de 0,1 à 0,6 % en poids.

12. Composition selon la revendication 1, comprenant en outre un médicament thérapeutique additionnel en mélange avec ledit étabonate de lotéprednol et ladite tobramycine, dans laquelle ledit médicament thérapeutique additionnel est choisi dans le groupe constitué par le bétaxolol, l'aténolol, le lévobunolol, l'épinéphrine, le dipivalyle, l'oxonolol, la base de l'acétazolamide, le méthazolamide, le piroxicam, l'indométhacine, la naproxène, la phénylbutazone, l'ibuprofène et l'acide diclofénac.

13. Composition selon la revendication 2, dans laquelle l'étabonate de lotéprednol est présent en une quantité comprise entre 0,01 et 1 % en poids.

14. Composition selon la revendication 2, dans laquelle l'étabonate de lotéprednol est présent en une quantité comprise entre 0,05 et 0,5 % en poids.

15. Composition selon la revendication 2, dans laquelle ledit polymère non ionique est un polymère hydrosoluble choisi dans le groupe constitué par la polyvinylpyrrolidone, un alcool polyvinylique, le dextran et la cyclodextrine.

16. Composition selon la revendication 2, dans laquelle le polymère non ionique est présent en une quantité de 0,2 à 2 % en poids.

17. Composition selon la revendication 15, dans laquelle ledit polymère non ionique est la polyvinylpyrrolidone et est présent en une quantité comprise entre 0,3 et 1,75 % en poids.

18. Composition selon la revendication 2, dans laquelle ledit agent tensioactif non ionique est le tyloxapol et est présent en une quantité de 0,05 à 1 % en poids.

19. Composition selon la revendication 2, dans laquelle ledit agent tensioactif non ionique est un polyester de monooléate de polyoxyéthylène sorbitan.

20. Composition selon la revendication 17, dans laquelle l'agent de tonicité non ionique est présent en une quantité comprise entre 1 et 7 % en poids.

21. Composition selon la revendication 20, dans laquelle l'agent de tonicité non ionique est un polyol non ionique et est présent en une quantité comprise entre 1,5 et 4 % en poids.

22. Composition selon la revendication 21, dans laquelle l'agent de tonicité non ionique est le glycérol ou le mannitol.

23. Composition selon la revendication 2, comprenant en outre un conservateur pour empêcher la formation microbienne dans ladite composition et qui est présent en une quantité comprise entre 0,0001 et 0,025 % en poids.

24. Composition selon la revendication 23, dans laquelle ledit conservateur est choisi dans le groupe constitué par le chlorure de benzalkonium, l'édétate de disodium et un de leurs mélanges.

25. Composition selon la revendication 2, comprenant en outre un médicament thérapeutique additionnel en mélange avec ledit étabonate de lotéprednol et ladite tobramycine, dans laquelle ledit médicament thérapeutique additionnel est choisi dans le groupe constitué par le bétaxolol, l'aténolol, le lévobunolol, l'épinéphrine, le dipivalyle, l'oxonolol, la base de l'acétazolamide, le méthazolamide, le piroxicam, l'indométhacine, le naproxène, la phénylbutazone, l'ibuprofène et le diclofénac.

26. Composition selon la revendication 2, dans laquelle le polymère non ionique est présent en une quantité comprise entre 0,2 et 2 % en poids ; l'agent de tonicité non ionique est présent en une quantité comprise entre 1 et 7 % en poids ; et l'agent tensioactif non ionique est présent en une quantité comprise entre 0,05 et 1 % en poids.

27. Composition selon la revendication 26, comprenant en outre un conservateur pour empêcher la formation microbienne dans ladite composition et est présent en une quantité de 0,0001 à 0,025 % en poids.

28. Utilisation d'une composition comprenant :
(A) de l'étabonate de lotéprednol en une quantité de 0,01 à environ 2 % en poids et ayant une taille de particule de 0,1 à 30 microns de diamètre ;
(B) une quantité de tobramycine efficace pour stabiliser le pH de la composition par rapport au pH d'une composition similaire sans tobramycine ;
(C) un polymère non ionique dans un milieu aqueux ; et
(D) un agent tensioactif non ionique en une quantité suffisante pour garder l'étabonate de lotéprednol en suspension, dans laquelle le rapport molaire de (A) : (C) : (D) est compris entre 1 : 20 : 1 et 1 ; 0,01 : 0,5 pour la fabrication d'un médicament destiné à traiter une inflammation et une infection ophtalmiques ou oto-rhino-laryngologiques.
